# EUROPÄISCHE PATENTSCHRIFT

(11) **EP 3 765 119 B1**
(45) Veröffentlichungstag und Bekanntmachung des Hinweises auf die Patenterteilung: **24.01.2024**
(21) Anmeldenummer: 19709907.0
(22) Anmeldetag: 07.03.2019
(51) Int. Cl.: A61M 1/36

(54) **VORRICHTUNG ZUR ÜBERWACHUNG DES GEFÄSSZUGANGS BEI EINEM EXTRAKORPORALEN BLUTBEHANDLUNG**
DEVICE FOR MONITORING THE VASCULAR ACCESS DURING AN EXTRACORPOREAL BLOOD TREATMENT
DISPOSITIF DE SURVEILLANCE D'UN ACCÈS VASCULAIRE EN COURS D'UN TRAITEMENT EXTRACORPOREL DU SANG

(30) Priorität: 16.03.2018 DE 102018106226
(43) Veröffentlichungstag der Anmeldung: 20.01.2021
(73) Patentinhaber: Fresenius Medical Care Deutschland GmbH, 61352 Bad Homburg v.d.H. (DE)
(72) Erfinder: HAUKE, Christopher, 55246 Mainz-Kostheim (DE); OFFERMANNS, Lars, 35510 Butzbach (DE)
(74) Vertreter: Oppermann, Frank
(86) Internationale Anmeldenummer: PCT/EP2019/055729
(87) Internationale Veröffentlichungsnummer: WO 2019/175021

(56) Entgegenhaltungen:
- WO-A1-2007/104350
- WO-A1-2016/023868

## Beschreibung

Die Erfindung betrifft eine Vorrichtung zur Überwachung eines Zuganges zu einem Patienten für eine Vorrichtung, mit der über eine Schlauchleitung eine Flüssigkeit von dem Patienten abgeführt wird und/oder dem Patienten zugeführt wird, insbesondere zur Überwachung des Gefäßzuganges bei einer extrakorporalen Blutbehandlung, bei der Blut eines Patienten über eine arterielle Schlauchleitung, die eine arterielle Punktionskanüle aufweist, von dem Patienten abgeführt und über eine venöse Schlauchleitung, die eine venöse Punktionskanüle aufweist, dem Patienten zugeführt wird. Darüber hinaus betrifft die Erfindung eine Blutbehandlungsvorrichtung mit einer Vorrichtung zur Überwachung eines Zuganges zu einem Patienten.

Auf dem Gebiet der Medizintechnik ist eine Vielzahl von Einrichtungen bekannt, mit denen über eine Schlauchleitung einem Patienten Flüssigkeiten zugeführt oder Flüssigkeiten von dem Patienten abgeführt werden können. Dabei erfolgt der Zugang zu dem Patienten im Allgemeinen mit einem Katheter zum Einführen in Körperorgane oder einer Kanüle oder Nadel zum Punktieren von Gefäßen. Während der Untersuchung oder Behandlung ist ein ordnungsgemäßer Zugang zu dem Patienten sicherzustellen. Daher ist es erforderlich, den Patientenzugang zu überwachen.

Ein Anwendungsfall mit besonders hohen Anforderungen an die Sicherheit des Gefäßzuganges stellt die extrakorporale Blutbehandlung dar, bei der über eine arterielle Schlauchleitung, die eine arterielle Punktionskanüle aufweist, Blut von dem Patienten abgeführt, das Blut durch einen Dialysator geleitet wird und über eine venöse Blutleitung, die eine venöse Punktionskanüle aufweist, dem Patienten wieder zugeführt wird. Dabei besteht trotz regelmäßiger Überwachung des Patientenzugangs durch das Krankenhauspersonal grundsätzlich die Gefahr, dass die venöse Punktionskanüle unbemerkt aus dem Blutgefäß des Patienten herausrutscht. Während ein Herausrutschen der arteriellen Kanüle mit einem Ansaugen von Luft in die arterielle Schlauchleitung verbunden ist, das aufgrund einer maschinenseitigen Luftdetektion zu einem Alarm und zur Unterbrechung der Behandlung führt, ist das Herausrutschen der venösen Kanüle und der damit gefürchtete Freifluss des Bluts in die Umgebung nicht ohne weiteres zu detektieren. Wenn das Herausrutschen der venösen Kanüle aber nicht sofort erkannt wird, kann der Patient verbluten.

Die standardmäßig in den Behandlungsmaschinen vorhandenen Sicherheitsvorrichtungen basieren im Allgemeinen auf einer Überwachung des Drucks im extrakorporalen Blutkreislauf. In der Praxis hat sich jedoch gezeigt, dass allein mit einer Überwachung des Drucks im extrakorporalen Blutkreislauf das Herausrutschen insbesondere der venösen Punktionskanüle nicht mit der ausreichenden Sicherheit erkannt werden kann.

Aus der WO 2007/104350 A1 und der WO 2013/0117252 sind einfach zu handhabende, kostengünstig herstellbare und jederzeit nachrüstbare Vorrichtung bekannt, die eine sichere Überwachung eines Patientenzugangs erlauben. Die Überwachung des Patientenzugangs beruht darauf, dass in der flüssigkeitsführenden Schlauchleitung eine Schlaufe gebildet wird. Es wird davon ausgegangen, dass ein Herausrutschen der Punktionskanüle auf das Angreifen von Zugkräften an der Schlauchleitung zurückzuführen ist. Wenn die Schlauchleitung auf Zug beansprucht wird, zieht sich die Schlaufe zwangsläufig zu. Dies führt zu einem erhöhten Druckverlust in der Schlauchleitung, der mit einem Drucksensor erkannt wird. Die WO 2007/104350 A1 und WO 2013/0117252 schlagen zum Fixieren der Schlauchleitung in Form einer Schlaufe die Verwendung von Fixierungselementen vor, mit denen ein Schlauchsegment der Schlauchleitung in Form einer Schlaufe derart fixiert ist, dass sich die Schlaufe unter Zugbeanspruchung zusammenziehen kann. Bei der WO 2007/104350 A1 erweist sich aber als nachteilig, dass die hierzu erforderlichen Zugkräfte relativ hoch sind. Daher kann bei nur geringen Zugkräften nicht sichergestellt werden, dass ein fehlerhafter Gefäßzugang erkannt wird. Zur Verringerung der Zugkräfte schlägt die WO 2013/0117252 ein Fixierungselement vor, das ein Führungsstück und ein Sicherungsstück umfasst.

Der Erfindung liegt die Aufgabe zugrunde, eine einfach zu handhabende, kostengünstig herstellbare und jederzeit nachrüstbare Vorrichtung zur Überwachung eines Zuganges zu einem Patienten zu schaffen, die einen fehlerhaften Gefäßzugang auch bei einer nur geringen Zugbeanspruchung der Schlauchleitung sicher erkennen lässt, sowie eine Blutbehandlungsvorrichtung mit einer derartigen Vorrichtung zur Überwachung eines Zuganges zu einem Patienten bereitzustellen.

Die Aufgabe der Erfindung betrifft sowohl Gefäßzugänge wie Fistel oder Shunt bei der chronischen extrakorporalen Blutbehandlung, die mittels Nadeln oder Kanülen punktiert werden, als auch Katheter, z.B. zentralvenöse Katheter bei der akuten extrakorporalen Blutbehandlung, z.B. Extracorporeal Critical Care Therapy (ECCT), insbesondere Continuous Renal Replacement Therapy (CRRT), sowie die Infusionstechnik.

Die Lösung dieser Aufgaben erfolgt erfindungsgemäß mit den Merkmalen der unabhängigen Patentansprüche. Vorteilhafte Ausführungsformen der Erfindung sind Gegenstand der Unteransprüche.

Die Lösung der Aufgabe betrifft sowohl Gefäßzugänge wie Fistel oder Shunt bei der chronischen extrakorporalen Blutbehandlung, die mittel Nadeln oder Kanülen punktiert werden, als auch zentralvenöse Katheter bei der akuten extrakorporalen Blutbehandlung, z.B. Extracorporeal Critical Care Therapy (ECCT), insbesondere Continuous Renal Replacement Therapy (CRRT), sowie die Infusionstechnik. Immer wenn von Nadel, Punktionskanüle oder Kanüle die Rede ist, gilt dies gleichermaßen auch für Katheter, z.B. zentralvenöse Katheter bei der akuten extrakorporalen Blutbehandlung.

Unter einem fehlerhaften Gefäßzugang wird nicht nur das völlige Herausrutschen der Punktionskanüle, sondern auch ein fehlerhafter Sitz der Kanüle verstanden, d. h. wenn die Punktionskanüle nur teilweise herausgerutscht ist.

Die erfindungsgemäße Vorrichtung zur Überwachung eines Patientenzuganges beruht darauf, dass ein Schlauchsegment der Schlauchleitung lose geführt wird. Hierfür findet eine Schlauchführung Verwendung, die Bestandteil der Schlauchleitung sein kann oder der Schlauchleitung lose beigelegt werden kann.

Für die Erfindung ist unerheblich, ob sich das lose geführte Schlauchsegment durch die Form eines Kreises auszeichnet, bei dem sich die Enden des Schlauchsegments überkreuzen, beispielsweise das Schlauchsegment eine Schlaufe oder eine Schlinge bildet, oder sich nur durch die Form eines gebogenen Abschnitts auszeichnet, deren Enden sich nicht überkreuzen. Die erfindungsgemäße Vorrichtung beruht nicht auf einer Überwachung einer Änderung des Drucks in der Schlauchleitung, die auf eine Erhöhung des Druckverlusts infolge einer Verringerung des Durchmessers der Schlaufe zurückzuführen ist, sondern auf der Erkennung einer Veränderung der Lage des Schlauchsegments infolge einer Zugbeanspruchung, wobei bei einer Lageänderung des Schlauchsegments auf einen fehlerhaften Gefäßzugang geschlossen wird. Da nur Lageänderungen überwacht werden, reichen auch schon geringfüge Zugbeanspruchungen aus, um auf einen fehlerhaften Gefäßzugang hinzuweisen. Die Lageänderung muss bei der erfindungsgemäßen Vorrichtung und dem erfindungsgemäßen Verfahren nicht zu einer Erhöhung des Druckverlusts im Schlauchsegment führen, um auf einen fehlerhaften Gefäßzugang hinzuweisen.

Die Erfindung sieht unterschiedliche Einrichtungen zur Lageerkennung des Schlauchsegments vor, die sich darin unterscheiden, dass die Lageerkennung berührungslos oder nicht berührungslos erfolgen kann. Den unterschiedlichen Einrichtungen zur Lageerkennung ist aber gemeinsam, dass sie Bestandteil der Schlauchführung sind. Die erfindungsgemäße Vorrichtung stellt somit eine einfach zu handhabende Einheit dar, die an der Schlauchleitung angebracht werden kann oder mit der Schlauchleitung bereits verbunden sein kann.

Unter der Lageerkennung eines Schlauchsegments wird in der vorliegenden Erfindung die Detektion jeder Lageänderung des Schlauchsegments oder jeder Positionsänderung des Schlauchsegments relativ zur Schlauchführung und/oder relativ zur Einrichtung zur Lageerkennung verstanden. Unter einer Lageänderung wird beispielsweise die Veränderung des Durchmessers der Schlaufe oder der Länge oder Breite des bogenförmigen Abschnitts verstanden. In manchen Ausführungsformen kann die Lageänderung des Schlauchsegments oder eines Abschnittes davon in axialer Richtung der zentralen Längsachse des Schlauchsegments erfolgen. In anderen Ausführungsformen kann die Lageänderung des Schlauchsegments oder eines Abschnittes davon von der axialen Richtung der zentralen Längsachse des Schlauchsegments abweichen.

Nach dem Punktieren der Fistel oder in des Shunts mittels der Kanüle oder der Nadel oder nach dem Legen eines zentralvenösen Katheters wird die Schlauchleitung frei von Zugkräften verlegt und das Schlauchsegment frei von Zugkräften durch die erfindungsgemäße Schlauchführung geführt, so dass eine Lageänderung des Schlauchsegment in einem bestimmten Umfang möglich ist, ohne dass eine unzulässige Zugkraft an der Kanüle oder Nadel oder dem zentralvenösen Katheter droht. Unter einer unzulässigen Lageänderung des Schlauchsegments wird in der vorliegenden Erfindung jede Lageänderung des Schlauchsegments oder jede Positionsänderung des Schlauchsegments relativ zur Schlauchführung und/oder relativ zur Einrichtung zur Lageerkennung verstanden, bei der die Gefahr einer unzulässigen Zugkraft an der Kanüle oder Nadel oder dem zentralvenösen Katheter droht, so dass das Herausziehen der Kanüle oder Nadel oder dem zentralvenösen Katheter aus dem Blutgefäßsystem des Patienten droht. In der Praxis sollten also nur geringfügige Lageänderungen der Schlauchleitung möglich sein.

Bei einigen Ausführungsformen weist die Einrichtung zur Lageerkennung ein optisches Bilderfassungssystem auf. Unter einem optischen Bilderfassungssystem wird in der vorliegenden Erfindung ein Erfassungssystem verstanden, das konfiguriert ist, eine Veränderung der räumlichen Lage einer Schlauchleitung mit Hilfe von Licht zu detektieren. Beispiele dafür sind Phototransistoren, z.B. zur Farberkennung und/oder hell-dunkel Erkennung und/oder Trübungserkennung, Sensoren zur Aufnahme von zweidimensionalen Lichtbildern wie CCD Sensoren und CMOS Sensoren.

In manchen Ausführungsformen ist das optische Bilderfassungssystem konfiguriert, eine Mehrzahl von beabstandeten Markierungen, die an der Schlauchleitung (7) angebracht sind, nacheinander einzeln zu detektieren und zu zählen, wenn die Markierungen aufgrund einer Lageänderung der Schlauchleitung relativ zum optischen Bilderfassungssystem bewegt werden und dessen Erfassungsbereich durchlaufen, wobei das optische Bilderfassungssystem eine Auswerteeinheit aufweist, die derart konfiguriert ist, dass die Anzahl der gezählten Markierungen mit einem vorgegebenen Grenzwert verglichen wird und auf eine Veränderung der Lage der Schlauchleitung infolge einer Beanspruchung der Schlauchleitung auf Zug geschlossen wird, wenn die Anzahl der gezählten Markierungen größer als ein vorgegebener Grenzwert ist.

Die Markierungen brauchen nicht über die gesamte Länge der Schlauchleitung, sondern nur an dem Schlauchsegment vorgesehen zu sein, das mit der Schlauchführung lose geführt wird. Die Markierungen können Streifen an der Schlauchleitung sein, die in einem vorgegebenen Abstand an der Schlauchleitung vorgesehen sind. Die Markierungen können beispielsweise auf die Schlauchleitung aufgeklebt oder aufgedruckt oder durch Beschichten aufgebracht sein.

Eine alternative Ausführungsform sieht vor, dass das optische Bilderfassungssystem derart konfiguriert ist, dass eine Mehrzahl von Markierungen, die an der Schlauchleitung vorgesehen sind, in einem Beobachtungsfenster bestimmt wird, wobei das optische Bilderfassungssystem eine Auswerteeinheit aufweist, die derart konfiguriert ist, dass die Anzahl der Markierungen in dem Beobachtungsfenster mit einem vorgegebenen Grenzwert verglichen wird und auf eine Veränderung der Lage der Schlauchleitung infolge einer Beanspruchung der Schlauchleitung auf Zug geschlossen wird, wenn die Anzahl der Markierungen kleiner als ein vorgegebener Grenzwert ist oder sich die Anzahl um eine vorgegebene Anzahl verringert. Das Beobachtungsfenster kann ein beliebiger Bildausschnitt sein, der von dem Bilderfassungssystem leicht erfasst werden kann. In dem Beobachtungsfenster sollte vorzugsweise der Abschnitt des Schlauchsegments liegen, der seine Lage bei einer Zugbeanspruchung besonders stark ändert, beispielsweise ein von der Schlauchführung nicht verdeckter Abschnitt des eine Schlaufe bildenden Schlauchsegments.

Das optische Bilderfassungssystem kann auch derart konfiguriert sein, dass der Abstand zwischen beliebigen Markierungen, die an der Schlauchleitung vorgesehen und in dem Beobachtungsfenster liegen, bestimmt wird, wobei das optische Bilderfassungssystem eine Auswerteeinheit aufweist, die derart konfiguriert ist, dass der Abstand zwischen den Markierungen in dem Beobachtungsfenster mit einem vorgegebenen Grenzwert verglichen wird und auf eine Veränderung der Lage der Schlauchleitung infolge einer Beanspruchung der Schlauchleitung auf Zug geschlossen wird, wenn der Abstand zwischen den Markierungen kleiner als ein vorgegebener Grenzwert ist.

Eine weitere Ausführungsform sieht vor, dass die Einrichtung zur Lageerkennung ein taktiles Sensorsystem aufweist, das derart konfiguriert ist, dass eine Veränderung der Lage der Schlauchleitung infolge einer Beanspruchung der Schlauchleitung auf Zug erkannt wird. Das taktile Sensorsystem kann einen Tastsensor aufweisen, der in federnder Vorspannung gegen das mit der Schlauchführung geführte Schlauchsegment gebracht werden kann.

Eine Ausführungsform eines taktilen Sensorsystems mit einem Tastsensor weist eine Auswerteeinheit auf, die das Messsignal eines Weglängenmessers empfängt, der derart ausgebildet ist, dass der Weg gemessen wird, um den sich der Tastsensor bei einer Veränderung der Lage des Schlauchsegments verschiebt. Die Auswerteeinheit kann derart konfiguriert sein, dass die gemessene Weglänge mit einem vorgegebenen Grenzwert verglichen wird und eine Veränderung der Lage der Schlauchleitung infolge einer Beanspruchung der Schlauchleitung auf Zug erkannt wird, wenn die gemessene Weglänge größer als ein vorgegebener Grenzwert ist oder sich um einen vorgegebenen Betrag verändert. Der Weglängenmesser kann jeder beliebige Messwertaufnehmer sein, der ein mit der Weglänge proportionales Signal erzeugt.

Eine alternative Ausführungsform sieht vor, dass der Tastsensor als Betätigungsorgan eines elektrischen Schalters ausgebildet ist. Bei dieser Ausführungsform führt die Zugbeanspruchung der Schlauchleitung zu einer Auslenkung des Tastsensors, wodurch der Schalter beispielsweise geschlossen wird, so dass in einem Stromkreis ein Strom fließt. Diese Ausführungsform zeichnet sich durch eine besonders einfache und kostengünstige Herstellung aus.

Die Einrichtung zur Lageerkennung kann auch ein langgestrecktes Betätigungselement aufweisen, das bei einer Verformung einen elektrischen Schalter betätigt. Bei dieser Ausführungsform steht das erste Ende des langgestreckten Betätigungselements mit einem ersten Abschnitt des mit der Schlauchführung geführten Schlauchsegments in Wirkverbindung, während das zweite Ende des langgestreckten Betätigungselements mit einem zweiten Abschnitt des mit der Schlauchführung geführten Schlauchsegments in Wirkverbindung steht. Da beide Enden des langgestreckten Betätigungselements mit unterschiedlichen Abschnitten des Schlauchsegments in Wirkverbindung stehen, wird das Betätigungselement bei einer Veränderung der Lage des Schlauchsegments infolge einer Zugbeanspruchung verformt, so dass der elektrische Schalter betätigt wird. Das Betätigungselement kann beispielsweise ein streifen- oder stabförmiges, vorzugsweise federelastisches Element aus Metall oder Kunststoff sein.

Die Einrichtung zur Lageerkennung kann auch ein langgestrecktes Element aus einem elektrisch leitenden Material aufweisen, wobei das erste Ende des elektrisch leitenden Elements mit einem ersten Abschnitt des mit der Schlauchführung geführten Schlauchsegments in Wirkverbindung steht, und das zweite Ende des elektrisch leitenden Elements mit einem zweiten Abschnitt des mit der Schlauchführung geführten Schlauchsegments in Wirkverbindung steht, so dass das langgestreckte Element bei einer Veränderung der Lage des Schlauchsegments infolge einer Beanspruchung auf Zug durchtrennt wird. Das langgestreckte Element kann ein einfacher elektrischer Draht sein, der bei einer Lageänderung des Schlauchsegments auf Zug beansprucht wird. Die Einrichtung zur Lageerkennung kann eine Auswerteeinheit aufweisen, die derart konfiguriert ist, dass der elektrische Widerstand des elektrisch leitenden Elements überwacht wird. Bei einer besonders einfach herzustellenden und kostengünstigen Ausführungsform kann der Draht Teil eines Stromkreises sein, der bei einer Lageänderung unterbrochen wird.

Darüber hinaus kann die Einrichtung zur Lageerkennung eine Laufrolle mit einem Umdrehungsmesser aufweisen, die mit dem in der Schlauchführung geführten Schlauchsegment in Kontakt gebracht werden kann, wobei eine das Messsignal des Umdrehungsmessers empfangende Auswerteeinheit vorgesehen ist, die derart konfiguriert ist, dass die Anzahl der Umdrehungen der Laufrolle mit einer vorgegebenen Grenzwert verglichen wird. Die Laufrolle mit dem Umdrehungsmesser erlaubt die Erkennung einer axialen Verschiebung eines Abschnitts des Schlauchsegments infolge einer Zugbeanspruchung. Gleichzeit kann die Laufrolle der besseren Führung des Schlauchsegments in der Schlauchführung dienen, so dass schon geringe Zugkräfte zu Lageänderungen führen können. Zur losen Führung des Schlauchsegments in der Schlauchführung können auch mehrere Laufrollen vorgesehen sein. Ein Umdrehungsmessers braucht aber dann nur bei einer der Laufrollen vorgesehen zu sein.

Eine Ausführungsform sieht eine Schlauchführung vor, die derart ausgebildet ist, dass das Schlauchsegment in Form einer Schlaufe geführt ist, die sich bei einer Zugbeanspruchung zusammenzieht. Diese Schlauchführung kann einen Gehäusekörper mit einem ersten Führungskanal zur Aufnahme eines ersten Abschnitts des Schlauchsegments und einem zweiten Führungskanal zur Aufnahme eines zweiten Abschnitts des Schlauchsegments der Schlauchleitung aufweisen, so dass das Schlauchsegment in der Form einer Schlaufe fixiert werden kann. Der Durchmesser zumindest eines der beiden Führungskanäle ist derart bemessen, dass ein Abschnitt des Schlauchsegments lose geführt ist, so dass sich die Schlaufe bei einer Zugbelastung zusammenziehen kann. Um das Schlauchsegment einfach in die Schlauchführung einlegen zu können, kann der Gehäusekörper zwei Gehäusehälften aufweisen, wobei die Führungskanäle in der ersten und/oder zweiten Gehäusehälfte des Gehäusekörpers ausgebildet sein können. Die erste und/oder zweite Gehäusehälfte des Gehäusekörper können beispielsweise lösbar oder öffenbar miteinander verbunden sind. Die lösbare Verbindung kann beispielsweise eine Rast- oder Schnappverbindung sein. Die beiden Gehäusehälften können auch mit Klammern oder dergleichen verbunden sein. Die öffenbare Verbindung kann eine Verbindung mit Scharnieren sein, so dass der Gehäusekörper aufklappbar ist. Der Gehäusekörper kann ein Spritzgussteil sein, wobei die Scharniere Filmscharniere sein können. Der Gehäusekörper kann aber auch aus nur einem Stück bestehen.

Bei einer alternativen Ausführungsform ist die Schlauchführung derart ausgebildet, dass das Schlauchsegment Form eines Bogens geführt ist, d. h. derart geführt ist, dass das Schlauchsegment nicht einen Kreis bildet. Bei dieser Ausführungsform kann die Schlauchführung einen Gehäusekörper aufweisen, in dem eine bogenförmige Anlagefläche für das Schlauchsegment ausgebildet ist, so dass das Schlauchsegment in Form eines Bogens fixiert ist. Eine Zugbeanspruchung der Schlauchleitung führt zu einer Verformung des Bogens, was eine Lageänderung des Schlauchsegments zur Folge hat.

Eine weitere Ausführungsform sieht vor, dass die Schlauchführung ein bogenförmiges Führungselement zur Aufnahme des Schlauchsegments aufweist. Bei dieser Ausführungsform kann das bogenförmige Führungselement einen ersten bogenförmigen Abschnitt zur Aufnahme eines ersten Teilstücks des Schlauchsegments und einen zweiten bogenförmigen Abschnitt zur Aufnahme eines zweiten Teilstücks des Schlauchsegments aufweisen, wobei der erste und zweite Abschnitt des Führungselements derart beweglich miteinander verbunden sind, dass sich der Abstand zwischen den gegenüberliegenden Enden der bogenförmigen Abschnitte des Führungselements vergrößert, wenn die Schlauchleitung auf Zug beansprucht wird. Das bogenförmige Führungselement zur Aufnahme des Schlauchsegments kann ein Profilstück sein, das derart ausgebildet ist, dass sich das Schlauchsegment in das Profilstück passend einlegen lässt. Das bogenförmige Führungselement kann auch aus einem elastischen Material bestehen, so dass sich der Abstand zwischen den gegenüberliegenden Enden der bogenförmigen Abschnitte des Führungselements vergrößert, wenn die Schlauchleitung auf Zug beansprucht wird. Bei dieser Ausführungsform ist eine bewegliche Verbindung zwischen zwei Teilstücken nicht erforderlich, so dass die Schlauchführung besonders einfach und kostengünstig hergestellt werden kann.

Die Einrichtung zur Lageerkennung kann derart konfiguriert sein, dass ein Steuersignal erzeugt wird, wenn eine Veränderung der Lage des Schlauchsegments infolge einer Beanspruchung der Schlauchleitung auf Zug erkannt und auf einen fehlerhaften Gefäßzugang geschlossen wird. In diesem Zusammenhang wird unter einem Steuersignal jedes Signal verstanden, dass einer weiteren Signalverarbeitung unterzogen werden kann.

Die erfindungsgemäße Vorrichtung zur Überwachung eines Zuganges zu einem Patienten kann insbesondere in einer Blutbehandlungsvorrichtung mit einem extrakorporalen Blutkreislauf verwendet werden, der eine arterielle Schlauchleitung mit einer arteriellen Punktionskanüle und eine venöse Schlauchleitung mit einer venösen Punktionskanüle aufweist. Die Blutbehandlungsvorrichtung kann eine Alarmeinheit aufweisen, die derart konfiguriert ist, dass ein Alarm gegeben wird, wenn die Einrichtung zur Lageerkennung eine Veränderung der Lage des Schlauchsegments infolge einer Beanspruchung der Schlauchleitung auf Zug erkennt. Bei der Blutbehandlungsvorrichtung, die eine Blutpumpe zum Fördern von Blut im extrakorporalen Blutkreislauf und ein venöses Absperrorgan zum Absperren der venösen Schlauchleitung aufweist, kann eine Steuereinheit vorgesehen sein, die derart konfiguriert ist, dass die Blutpumpe angehalten wird und/oder das venöse Absperrorgan geschlossen wird, wenn die Einrichtung zur Lageerkennung eine Veränderung der Lage des Schlauchsegments erkennt.

Zur drahtlosen Übertragung eines Steuersignals kann die Einrichtung zur Überwachung des Gefäßzugangs einen Sender und die Blutbehandlungsvorrichtung einen Empfänger aufweisen. Die Signalübertragung kann mit optischen Signalen und/oder elektrischen Signalen (Funk) erfolgen. Es ist aber auch möglich, dass die Einrichtung zur Überwachung des Gefäßzugangs und die Blutbehandlungsvorrichtung mit einem elektrischen Verbindungskabel miteinander verbunden sind.

Im Folgenden werden verschiedene Ausführungsbeispiele der Erfindung unter Bezugnahme auf die Zeichnungen näher erläutert.

Es zeigen:
- Fig. 1: die wesentlichen Komponenten einer Blutbehandlungsvorrichtung zusammen mit der erfindungsgemäßen Vorrichtung zur Überwachung des Patientenzugangs in stark vereinfachter schematischer Darstellung,
- Fig. 2: ein erstes Ausführungsbeispiel der erfindungsgemäßen Vorrichtung zur Überwachung des Patientenzugangs, bei dem die Lageänderung der Schlauchleitung optisch erfasst wird,
- Fig. 3: eine Teilansicht einer alternativen Ausführungsform des ersten Ausführungsbeispiels der erfindungsgemäßen Vorrichtung,
- Fig. 4: ein weiteres Ausführungsbeispiel der erfindungsgemäßen Vorrichtung zur Überwachung des Patientenzugangs, bei dem die Lageänderung der Schlauchleitung optisch erfasst wird,
- Fig. 5: das Ausführungsbeispiel von Fig. 4 in der Frontansicht, wobei die Schlauchführung geöffnet ist,
- Fig. 6: ein weiteres Ausführungsbeispiel der erfindungsgemäßen Vorrichtung zur Überwachung des Patientenzugangs, bei dem die Lageänderung der Schlauchleitung optisch erfasst wird,
- Fig. 7: ein weiteres Ausführungsbeispiel der erfindungsgemäßen Vorrichtung zur Überwachung des Patientenzugangs, bei dem die Lageänderung der Schlauchleitung mittels Messung der Umdrehungen mindestens einer Laufrolle erfasst wird,
- Fig. 8: ein weiteres Ausführungsbeispiel der erfindungsgemäßen Vorrichtung zur Überwachung des Patientenzugangs, bei dem die Lageänderung der Schlauchleitung taktil erfasst wird,
- Fig. 9: eine alternative Ausführungsform der erfindungsgemäßen Vorrichtung von Fig. 8,
- Fig. 10: ein weiteres Ausführungsbeispiel der erfindungsgemäßen Vorrichtung zur Überwachung des Patientenzugangs,
- Fig. 11: eine alternative Ausführungsform der erfindungsgemäßen Vorrichtung von Fig. 10 und
- Fig. 12: ein weiteres Ausführungsbeispiel der erfindungsgemäßen Vorrichtung zur Überwachung des Patientenzugangs.

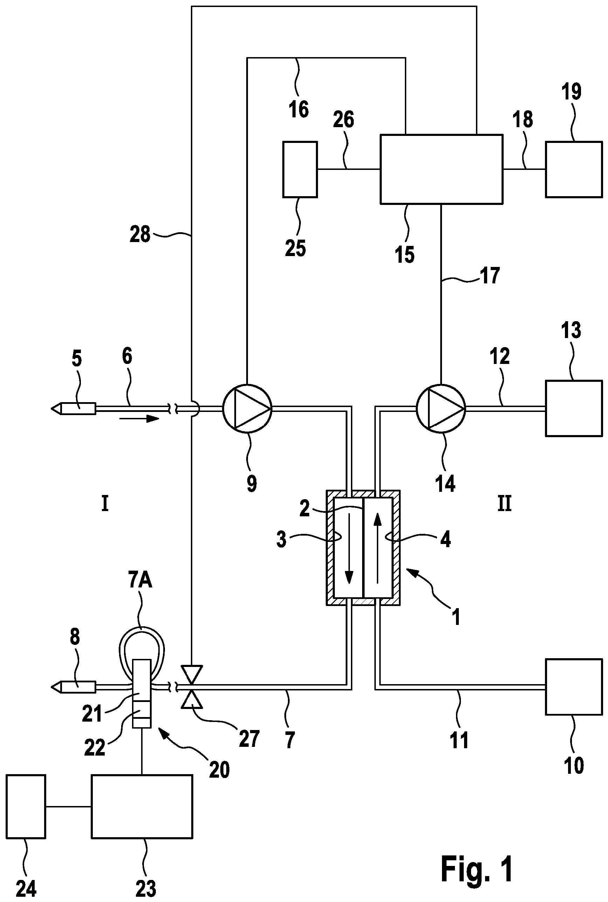

Fig. 1 zeigt die wesentlichen Komponenten einer Blutbehandlungsvorrichtung, beispielsweise eine Hämodialysevorrichtung, die über eine Vorrichtung zur Überwachung des venösen Gefäßzugangs verfügt. Die Blutbehandlungsvorrichtung weist einen Dialysator 1 auf, der durch eine semipermeable Membran 2 in eine Blutkammer 3 und eine Dialysatkammer 4 unterteilt ist. An der Fistel oder dem Shunt des Patienten ist mittels einer arteriellen Punktionskanüle 5 eine arterielle Schlauchleitung 6 angeschlossen, die zu dem Einlass der Blutkammer 3 des Dialysators 1 führt. Von dem Auslass der Blutkammer 3 des Dialysators 1 geht eine venöse Schlauchleitung 7 ab, die mittels einer venösen Punktionskanüle 8 an der Fistel oder dem Shunt des Patienten angeschlossen ist. Die arterielle Schlauchleitung 6 ist in eine okkludierende Blutpumpe 9 eingelegt, die das Blut im extrakorporalen Blutkreislauf I fördert.

Der Dialysatkreislauf II der Blutbehandlungsvorrichtung umfasst eine Dialysatquelle 10, an der eine Dialysatzuführleitung 11 angeschlossen ist, die zu dem Einlass der Dialysatkammer 4 des Dialysators führt. Von dem Auslass der Dialysatkammer 4 des Dialysators 1 geht eine Dialysatabführleitung 12 ab, die zu einem Auslass 13 führt. In die Dialysatabführleitung 12 ist eine Dialysatpumpe 14 geschaltet.

Die Steuerung der Blutbehandlungsvorrichtung übernimmt eine zentrale Steuereinheit 15, die über Steuerleitungen 16, 17 die Blut- und Dialysatpumpe 9, 14 ansteuert. Die zentrale Steuereinheit 15 ist über eine Datenleitung 18 mit einer Alarmeinheit 19 verbunden, die bei einem Störfall einen optischen und/oder akustischen und/oder taktilen Alarm gibt.

Die Blutbehandlungsvorrichtung weist eine Vorrichtung 20 zur Überwachung des venösen Gefäßzugangs auf. Die Überwachungsvorrichtung 20 weist eine in Fig. 1 nur schematisch dargestellte Schlauchführung 21 auf, die ein Schlauchsegment 7A der venösen Schlauchleitung 7 lose in einer bestimmten Form, beispielsweise in Form einer Schlaufe fixiert. Darüber hinaus weist die Überwachungsvorrichtung 20 eine in Fig. 1 nur schematisch dargestellte Einrichtung 22 zur Lageerkennung des mit der Schlauchführung 21 geführten Schlauchsegments 7A und eine Auswerteeinheit 23 auf. Die Auswerteeinheit 23 erzeugt ein Steuersignal, wenn -eine Lageänderung des Schlauchsegments 7A detektiert wird. Die Überwachungsvorrichtung 20 weist ferner einen Sender 24 auf, der das Steuersignal aussendet. Die Blutbehandlungsvorrichtung weist einen Empfänger 25 auf, der das Steuersignal empfängt. Der Empfänger 25 ist über eine Datenleitung 26 mit der zentralen Steuereinheit 15 der Blutbehandlungsvorrichtung verbunden.

An der venösen Schlauchleitung 7 befindet sich stromab der Blutkammer 3 des Dialysators ein Absperrorgan 27, beispielsweise eine elektromagnetisch betätigbare venöse Schlauchklemme, die über eine weitere Steuerleitung 28 von der zentralen Steuereinheit 15 geschlossen wird, wenn der Empfänger 25 das Steuersignal der Überwachungsvorrichtung 20 empfängt, das eine unzulässige Lageänderung des Schlauchsegments 7A signalisiert. In diesem Fall stoppt die Steuereinheit 15 die Blutpumpe 9 und/oder schließt das Absperrorgan 27, insbesondere die venöse Schlauchklemme.

Nachfolgend werden verschiedene Ausführungsbeispiele der Überwachungsvorrichtung 20 im Einzelnen beschrieben.

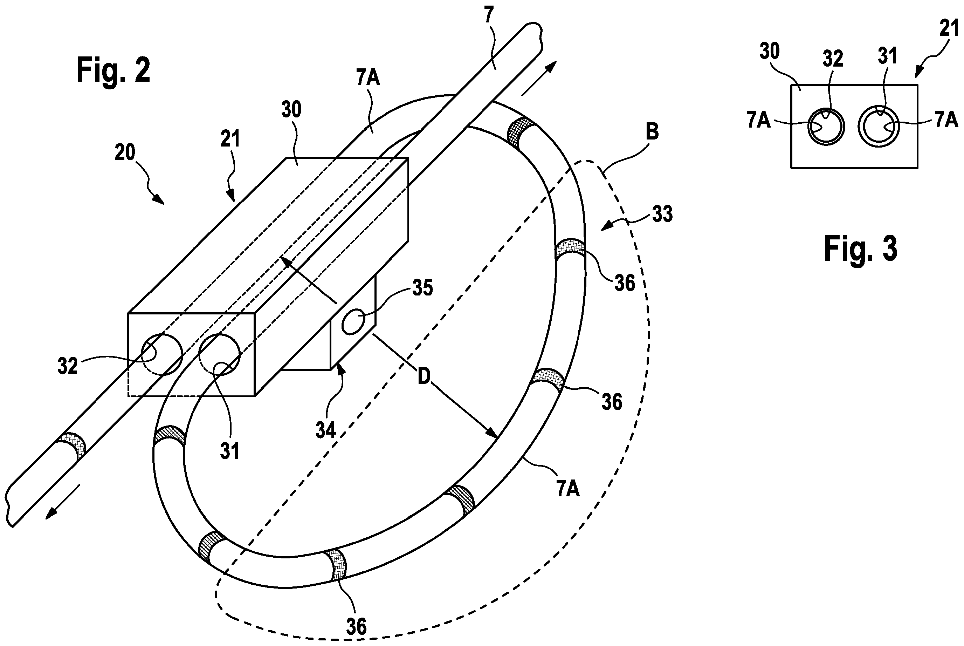

Fig. 2 zeigt in einer perspektivischen Darstellung ein erstes Ausführungsbeispiel der Überwachungsvorrichtung 20, die über ein optisches Bilderfassungssystem verfügt. Die Schlauchführung 21 weist einen im Spritzgussverfahren hergestellten Gehäusekörper 30 auf, der zwei parallel zueinander verlaufende Führungskanäle 31, 32 hat, deren Innendurchmesser größer als der Außendurchmesser der Schlauchleitung 7 sind. In den beiden Führungskanälen 31, 32 sind die beiden endseitigen Abschnitte eines eine Schlaufe 33 bildendenden Schlauchsegments 7Ader Schlauchleitung 7 lose geführt. Wenn auf die Enden der Schlauchleitung eine Zugkraft ausgeübt wird, was in Figur 1 durch Pfeile dargestellt ist, zieht sich die Schlaufe 33 zusammen. Der Gehäusekörper 30 der Schlauchführung 21 kann unterschiedlich geformt sein, beispielweise in Form eines Quaders oder in einer Bogenform.

Fig. 3 zeigt eine Frontansicht der Schlauchführung 21 einer alternativen Ausführungsform, die sich von dem Ausführungsbeispiel von Fig. 2 dadurch unterscheidet, dass nur ein Abschnitt des Schlauchsegments 7A in einem Führungskanal 31 lose geführt ist, so dass sich die Schlaufe 33 nur dann zusammenzieht, wenn an dem lose geführten Schlauchabschnitt gezogen wird.

Wenn sich die Schlaufe 33 bei einer Zugbeanspruchung zusammenzieht, verändert sich die Lage des in der Schlauchführung 21 geführten Schlauchsegments 7A, wobei sich der Durchmesser D des Schlauchsegments verringert. Die Einrichtung 22 zur Lageerkennung des Schlauchsegments weist ein optisches Bilderfassungssystem 34 auf, das eine die Bilddaten auswertende Auswerteeinheit 23 (Fig. 1) aufweist. Das optische Bilderfassungssystem 34, das sich in dem Gehäusekörper 30 befindet, weist einen optischen Sensor 35 auf, beispielsweise ein CCD-Sensor, der einen Bildbereich erfasst, in dem die Schlaufe 33 liegt. Zur Erkennung einer Lagevänderung des Schlauchsegments 7A können die dem Fachmann bekannten Bildverarbeitungsverfahren herangezogen werden.

Zur Vereinfachung der Lageerkennung mit dem optischen Bilderfassungssystem kann die Schlauchleitung mit Markierungen 36 beispielsweise in Form von Streifen versehen sein. Die Streifen, die eine vorgegebene Breite haben und in einem vorgegebenen Abstand zueinander angeordnet sind, können von dem Bilderfassungssystem 34 leicht erkannt werden. Beim Zusammenziehen der Schlaufe 33, d. h. einer Lageänderung des Schlauchsegments 7A, verringert sich der Abstand zwischen den einzelnen Markierungen. Darüber hinaus nimmt die Anzahl der Markierungen 36 in Bezug auf ein vorgegebenes Beobachtungsfenster B ab, das von dem Bildbereich oder einem Teilbereich des Bildbereichs bestimmt wird, der von dem optischen Sensor 35 erfasst wird.

Die Auswerteeinheit 23 ist derart konfiguriert, dass der Abstand zwischen zwei beliebigen Markierungen einer Mehrzahl von Markierungen 36 bestimmt und mit einem vorgegebenen Grenzwert verglichen wird und auf eine Veränderung der Lage der Schlauchleitung geschlossen wird, wenn der Abstand zwischen den Markierungen 36 kleiner als ein vorgegebener Grenzwert ist oder sich um einen bestimmten Betrag verringert. Alternativ oder zusätzlich kann die Auswerteeinheit 23 derart konfiguriert sein, dass die Anzahl der Markierungen 36 in dem Beobachtungsfenster B bestimmt und mit einem vorgegebenen Grenzwert verglichen wird und auf eine Veränderung der Lage der Schlauchleitung geschlossen wird, wenn die Anzahl der Markierungen 36 kleiner als ein vorgegebener Grenzwert ist oder sich um eine bestimmte Anzahl verringert.

Anstelle eines optischen Bilderfassungssystems 34 kann in den Gehäusekörper der Schlauchführung auch ein Abstandssensor, beispielsweise ein optischer Abstandssensor oder ein Ultraschall-Abstandssensor, integriert sein, der den Abstand zu dem gegenüberliegenden Abschnitt des Schlauchsegments 7A misst. Die Auswerteeinheit 23 ist derart konfiguriert, dass der gemessene Abstandswert mit einem vorgegeben Grenzwert verglichen wird. Wenn der Abstandswert kleiner als der Grenzwert ist oder sich um einen bestimmten Betrag verringert, wird das Steuersignal erzeugt.

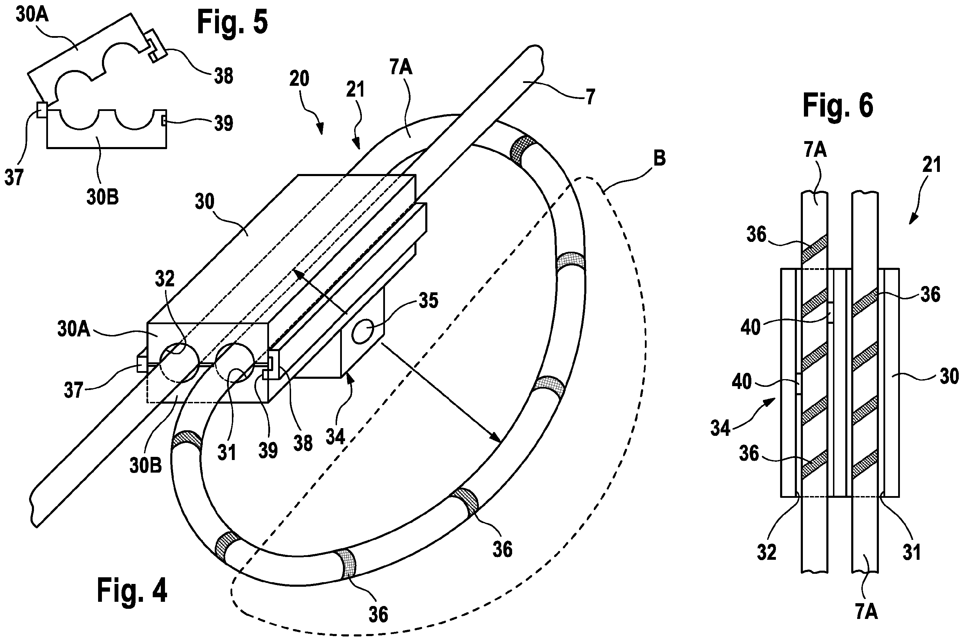

Die Figuren 4 und 5 zeigen eine Ausführungsform der Überwachungsvorrichtung 20, die sich von dem Ausführungsbeispiel von Figur 2 dadurch unterscheidet, dass der Gehäusekörper 30 der Schlauchführung 21 eine erste Gehäusehälfte 30A und eine zweite Gehäusehälfte 30B aufweist, die an einer Längsseite mit einem Scharnier 37 miteinander verbunden sind, so dass der Gehäusekörper 30 aufklappbar ist. Die einander entsprechenden Teile sind mit denselben Bezugszeichen versehen. Fig. 4 zeigt den zusammengeklappten Gehäusekörper 30 in perspektivischer Darstellung, während Fig. 5 den aufgeklappten Gehäusekörper 30 in der Frontansicht zeigt. Diese Ausführungsform hat den Vorteil, dass das Schlauchsegment 7A in die Schlauchführung 21 leicht eingelegt werden kann. Zum Verschließen des Gehäusekörpers 30 ist an einer Längsseite der oberen Gehäusehälfte 30A eine Klammer 38 vorgesehen, die klemmend in eine Nut 39 an der unteren Gehäusehälfte 30B greift.

Fig. 6 zeigt eine weitere Ausführungsform der Überwachungsvorrichtung 20, die sich in dem Bilderfassungssystem 34 von der Ausführungsform von Figur 2 unterscheidet. Das Bilderfassungssystem 34 der Ausführungsform von Fig. 6 weist nicht einen außerhalb des Gehäusekörpers 30 befindlichen optischen Sensor (Fig. 2), sondern einen oder mehrere optische Sensoren 40 auf, die innerhalb des Gehäusekörpers 30 an einem oder beiden Führungskanälen 31, 32 der Schlauchführung 21 vorgesehen sind. Die optischen Sensoren 40 erfassen die Markierungen 36 an dem Schlauchsegment 7A, beispielsweise in Form von Streifen, die eine vorgegebene Breite haben und in einem vorgegebenen Abstand zueinander angeordnet sind. Ein einziger optische Sensor 40 ist für die Erfassung der Markierungen ausreichend. Der optische Sensor kann beispielsweise ein Phototransistor sein, der die Markierungen beispielsweise durch eine hell-dunkel Erkennung erkennt. Wenn sich die Schlaufe 33 zusammenzieht, wird der betreffende Abschnitt des Schlauchsegments in dem Führungskanal 32 in Längsrichtung verschoben. Die Auswerteeinheit 23 des Bilderfassungssystems 34 kann derart konfiguriert sein, dass die Lageänderung der Markierungen 36 erkannt und bei einer Zugbeanspruchung das Steuersignal erzeugt wird. Die Auswerteeinheit 23 kann auch derart konfiguriert sein, dass die Anzahl der Markierungen 36 gezählt wird, wenn die Markierungen 36 aufgrund einer Lageänderung der Schlauchleitung (7) relativ zu dem optischen Sensor 40 des Bilderfassungssystems bewegt werden und dessen Erfassungsbereich durchlaufen, und die Anzahl der Markierungen 36 mit einem vorgegebenen Grenzwert verglichen wird. Wenn die Anzahl der Markierungen 36 größer als der Grenzwert ist, wird das Steuersignal erzeugt. Da Breite und Abstand der streifenförmigen Markierungen 36 bekannt ist, kann die Auswerteeinheit 23 die Wegstrecke berechnen, um die sich die Schlaufe 33 zusammenzieht. Die Markierungen können auch aus Materialen bestehen, die nicht optisch erfasst werden können, beispielsweise Markierungen, die eine Veränderung des Magnetfeldes, der Induktivität oder der Kapazität bewirken. Anstelle von optischen Sensoren weist die Einrichtung zur Lageerkennung dann Sensoren zur Erfassung dieser physikalischen Größen auf.

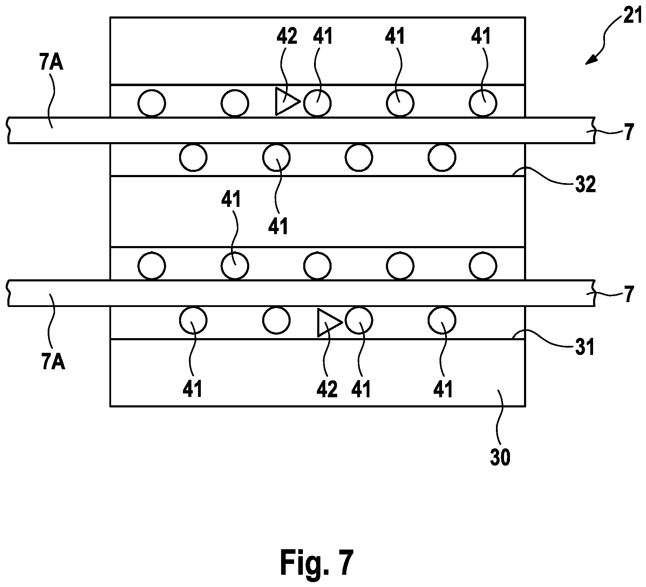

Fig. 7 zeigt eine alternative Ausführungsform, bei der in dem Gehäusekörper 30 der Schlauchführung 21, welcher gemäß Fig. 2 oder Fig. 4 ausgebildet sein kann, entlang mindestens einer der Führungskanäle 31, 32 Laufrollen 41 vorgesehen sind, die auf dem betreffenden Abschnitt des Schlauchsegments 7A bei dessen Lageänderung abrollen können, so dass sich die Schlaufe 33 besonders leicht zusammenziehen kann. Die Einrichtung 22 zur Lageerkennung weist bei dieser Ausführungsform einen in Fig. 7 nur schematisch dargestellten Umdrehungsmesser 42 auf, der einer der Laufrollen 41 zugeordnet ist, so dass die Anzahl der Umdrehungen der Laufrolle erfasst werden kann. Darüber hinaus ist eine das Messsignal des Umdrehungsmessers 42 empfangende Auswerteeinheit 23 vorgesehen, die derart konfiguriert ist, dass die Anzahl der Umdrehungen der Laufrolle 41 mit einem vorgegebenen Grenzwert verglichen wird. Das Steuersignal wird erzeugt, wenn die Anzahl der Umdrehungen größer als der Grenzwert ist.

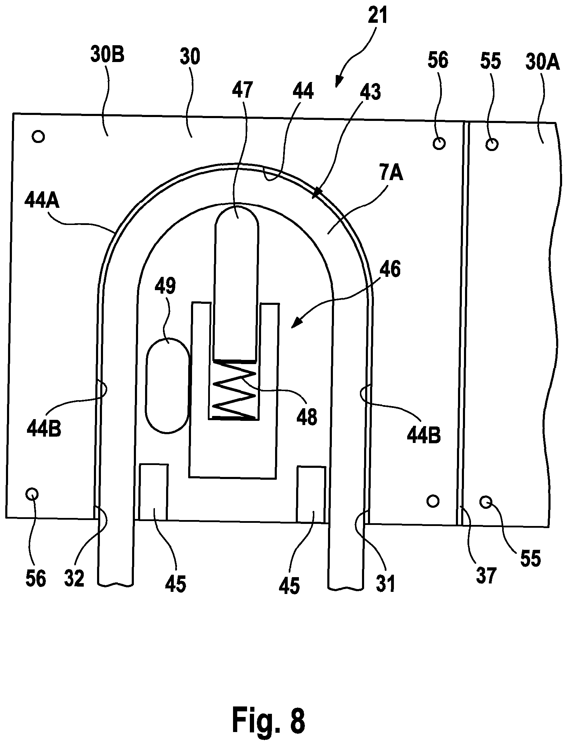

Unter Bezugnahme auf die Figuren 8 bis 12 werden nachfolgend weitere Ausführungsformen beschrieben, bei denen die Lageänderung nicht mit optischen Mitteln erkannt wird. Das Schlauchsegment 7A ist bei den Ausführungsformen der Figuren 8 bis 12 in Form eines Bogens 43 fixiert. Es ist aber auch möglich das Prinzip der Erkennung einer Lageänderung auf die anderen Ausführungsformen zu übertragen, bei denen das Schlauchsegment 7A eine Schlaufe 33 bildet.

Die Schlauchführung 21 von Fig. 8 weist einen Gehäusekörper 30 mit einer ersten und einer zweiten Gehäusehälfte auf, die mit einem Scharnier 37 miteinander verbunden sind, so dass die Gehäusehälften 30A, 30B zusammenklappbar sind. An einer Gehäusehälfte 30A können Rastnasen 55 vorgesehen sein, die in Ausnehmungen 56 an der anderen Gehäusehälfte 30B greifen. Die eine Gehäusehälfte 30B weist eine bogenförmige Anlagefläche 44 mit einem halbkreisförmigen Abschnitt 44A und zwei parallelen geradlinigen Abschnitten 44B auf, an die sich das Schlauchsegment 7A anlegen lässt, so dass das Schlausegment lose in Form eines Bogens 43 fixiert ist. Zur weiteren Führung des Schlauchsegments 7A können weitere Führungselemente 45 vorgesehen sein, die insbesondere in den Bereichen angeordnet sein können, an denen sich die Schlauchleitungsabschnitte aus dem Gehäusekörper 30 erstrecken.

Die Einrichtung 22 zur Lageerkennung verfügt über ein taktiles Sensorsystem 46, das einen axial verschiebbar geführten Tastsensor 47 aufweist, der mit einer Feder 48 gegen den halbkreisförmigen Abschnitt 44A des Schlauchsegments 7A vorgespannt ist, so dass das Schlauchsegment gegen den halbkreisförmigen Abschnitt gedrückt wird. Darüber hinaus weist die Einrichtung 22 zur Lageerkennung einen Weglängenmessers 49 auf, der in Fig. 8 nur schematisch dargestellt ist, und eine Auswerteeinheit 23 auf, die das Messsignal des Weglängenmessers 49 empfängt. Die Auswerteeinheit 23 ist derart ausgebildet, dass der Weg gemessen wird, um den sich der Tastsensor 47 bei einer Veränderung der Lage des Schlauchsegments infolge einer Zugbeanspruchung der Schlauchleitung verschiebt. Die Auswerteeinheit 23 vergleicht die gemessene Weglänge mit einem vorgegebenen Grenzwert und erzeugt das Steuersignal, wenn die gemessene Weglänge größer als ein vorgegebener Grenzwert ist.

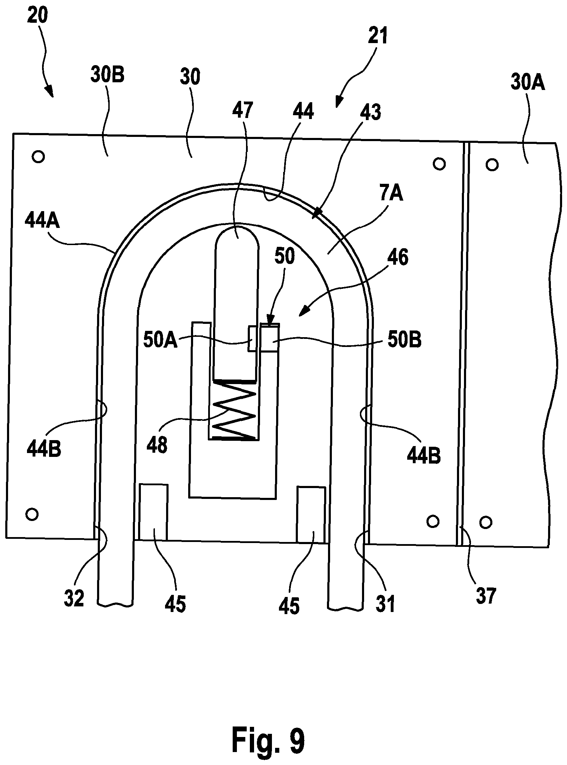

Fig. 9 zeigt eine alternative Ausführungsform, die sich von dem Ausführungsbeispiel von Fig. 8 dadurch unterscheidet, dass der Tastsensor 47 als Betätigungsorgan eines elektrischen Schalters 50 ausgebildet ist. Der Schalter 50 hat einen ersten beweglichen Schaltkontakt 50A, der an dem Tastsensor 47 vorgesehen ist, und eine zweiten ortfesten Schaltkontakt 50B, der an dem Gehäusekörper 30 vorgesehen ist. Bei einer Verschiebung des Tastsensors 47 infolge einer Zugbeanspruchung öffnet sich der Schalter 50. Bei dieser Ausführungsform kann mit dem Schalter 50 bei einer unzulässigen Lageänderung des Schlauchsegments 7A beispielsweise eine Alarmeinheit 19 (Fig. 1), die auch an der Überwachungsvorrichtung 21 selbst vorgesehen sein kann, eingeschaltet werden, ohne dass eine weitere Signalverarbeitung erforderlich ist.

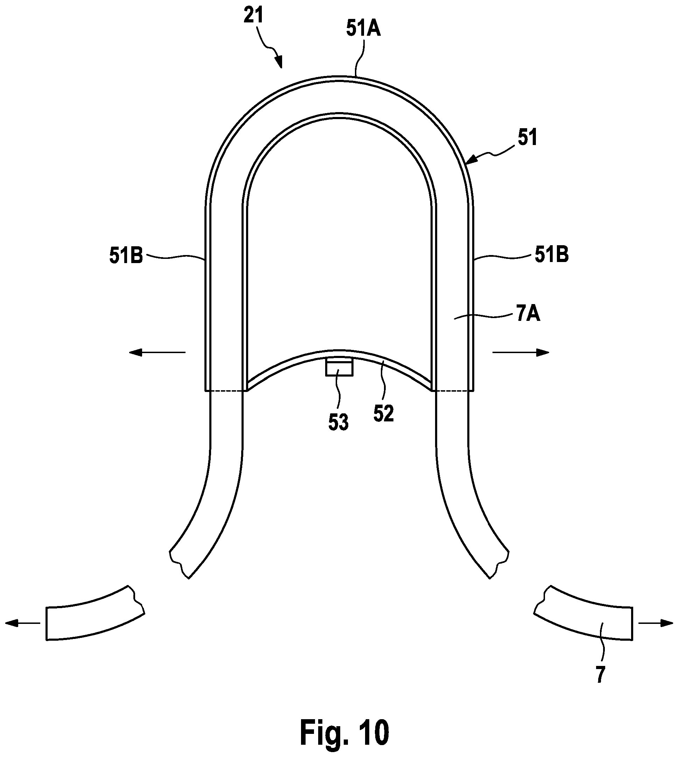

Fig. 10 zeigt eine Ausführungsform, bei der die Schlauchführung 21 ein bogenförmiges Führungselement 51 aufweist, in das das Schlauchsegment 7A passend eingelegt werden kann. Bei dem vorliegenden Ausführungsbeispiel ist das bogenförmige Führungselement 51 eine gebogene im Wesentlichen U-förmige Schiene mit einem halbkreisförmigen Abschnitt 51A und zwei parallelen geradlinigen Abschnitten 51B. Das bogenförmige Führungselement 51 besteht aus einem elastischen Material, beispielsweise Kunststoff, so sich das Führungselement verbiegen kann. Wenn die Schlauchleitung 7 auf Zug beansprucht wird, vergrößert sich der Abstand zwischen den gegenüberliegenden äußeren Enden des Führungselements 51, was in Fig. 10 durch Pfeile dargestellt ist. Das Führungselement 51 kann ein Kunststoff-Profil sein, das das Schlauchsegment teilweise umschließt.

Die Einrichtung 22 zur Lageerkennung weist ein langgestrecktes, streifenförmiges Element 52 auf, dessen eines Ende mit dem einen geradlinigen Abschnitt 51B und dessen anderes Ende mit dem anderen geradlinigen Abschnitt 51B des Führungselements 51 verbunden ist, wobei das streifenförmige Element 52 vorzugsweise im Bereich der Enden des Führungselements befestigt ist. Das streifenförmige Element 52, das ein Metallstreifen sein kann, ist federnd in eine gebogene Form vorgespannt. Bei einer Zugbeanspruchung der Schlauchleitung 7 wird zumindest einer der beiden geradlinigen Abschnitte 51B des Führungselements 51 nach außen verbogen, so dass sich das streifenförmige Element 52 entgegen der Vorspannung verformt und nicht mehr gebogen ist. Wenn sich das streifenförmige Element 52 verformt, wird ein nur schematisch dargestellter Schalter 53 betätigt. Anstelle eines Schalters kann aber an dem streifenförmigen Element auch ein in Fig. 10 nicht dargestellter Dehnungsmesstreifen vorgesehen sein.

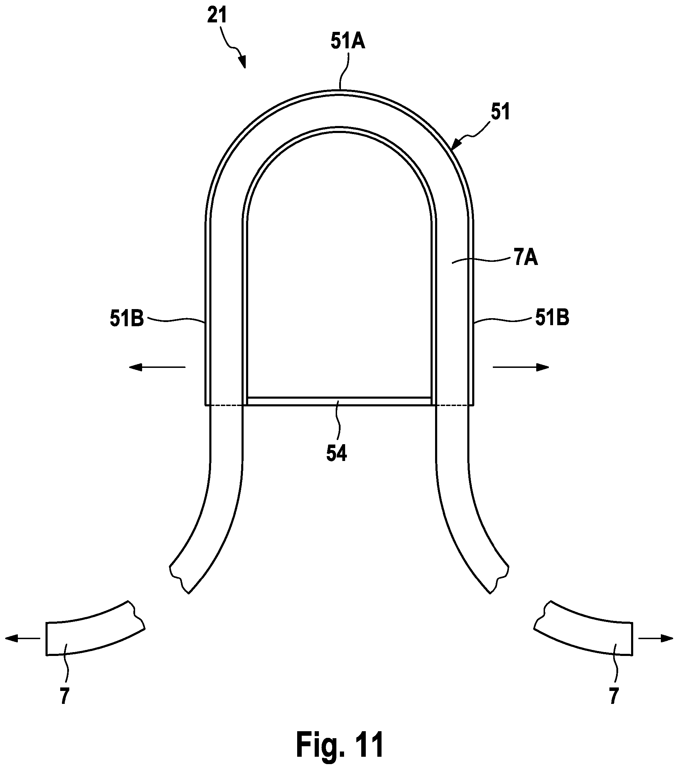

Fig. 11 zeigt eine alternative Ausführungsform, die sich von dem Ausführungsbeispiel von Fig. 10 dadurch unterscheidet, dass die Einrichtung 22 zur Lageerkennung ein langgestrecktes Element 54 aus einem elektrisch leitenden Material aufweist, das an den Enden der geradlinigen Abschnitte 51B des Führungselements 51 befestigt ist. Bei dem vorliegenden Ausführungsbeispiel ist das langgestreckte Element 54 ein elektrischer Draht, der durchtrennt wird, wenn sich bei einer Veränderung der Lage des Schlauchsegments 7A infolge einer Zugbeanspruchung zumindest einer der beiden geradlinigen Abschnitte 51B des Führungselements 51 nach außen biegt. Die Einrichtung 22 zur Lageerkennung weist bei der alternativen Ausführungsform eine Auswerteeinheit 23 auf, die derart konfiguriert ist, dass der elektrische Widerstand des Drahtes überwacht wird. Wenn der Widerstand null ist, wird das Steuersignal erzeugt.

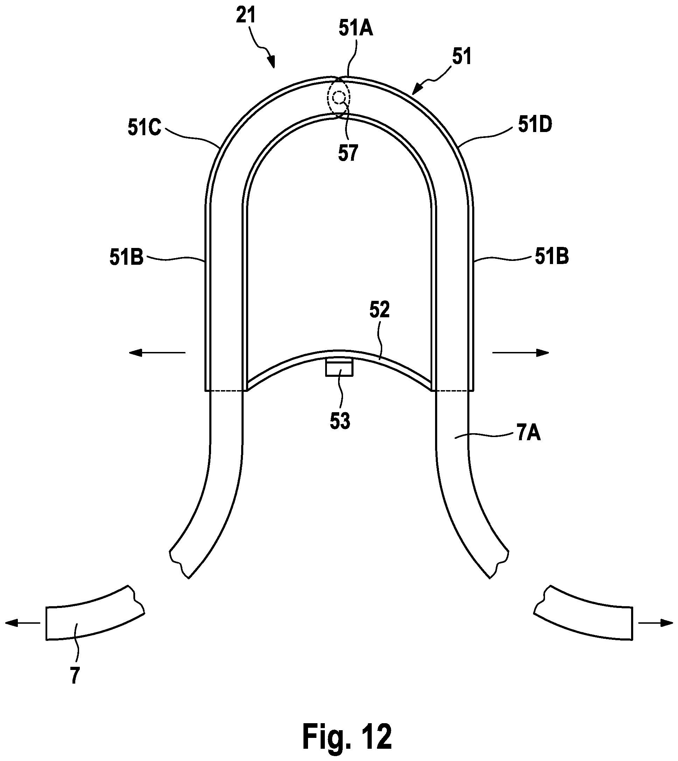

Fig. 12 zeigt eine alternative Ausführungsform, die sich von dem Ausführungsbeispiel von Fig. 10 dadurch unterscheidet, dass das Führungselement 51 einen ersten bogenförmigen Abschnitt 51C zur Aufnahme eines ersten Teilstücks des Schlauchsegments 7A und einen zweiten bogenförmigen Abschnitt 51D zur Aufnahme eines zweiten Teilstücks des Schlauchsegments 7A aufweist. Die einander entsprechenden Teile sind wieder mit denselben Bezugszeichen versehen. Der erste und zweite Abschnitt 51C, 51D des Führungselements 51 sind an den Enden mittels eines Gelenkes 57 beweglich miteinander verbunden, so dass sich der Abstand zwischen den gegenüberliegenden freien Enden des Führungselements vergrößert, wenn die Schlauchleitung auf Zug beansprucht wird, was wieder durch Pfeile dargestellt ist. Bei dieser Ausführungsform können die Teilstücke aus einem nicht elastischen Material bestehen. Bei der Ausführungsform von Fig. 12 kann anstelle eines elastischen streifenförmigen Elements 52 wie bei Fig. 10, insbesondere eines Metallstreifens zur Betätigung eines Schalters 53, auch ein Dehnungsmessstreifen oder ein elektrischer Draht 54 (Fig. 11) vorgesehen sein.

## Patentansprüche

1. Vorrichtung zur Überwachung eines Zuganges zu einem Patienten für eine Vorrichtung, mit der über eine Schlauchleitung (7) eine Flüssigkeit von dem Patienten abgeführt wird und/oder dem Patienten zugeführt wird, insbesondere zur Überwachung des Gefäßzuganges bei einer extrakorporalen Blutbehandlung, bei der Blut eines Patienten über eine arterielle Schlauchleitung, die eine arterielle Punktionskanüle aufweist, von dem Patienten abgeführt und über eine venöse Schlauchleitung, die eine venöse Punktionskanüle aufweist, dem Patienten zugeführt wird,
**dadurch gekennzeichnet, dass**
die Vorrichtung (20) zur Überwachung eines Zuganges zu einem Patienten eine Schlauchführung (21) zum Führen eines Schlauchsegments (7A) der Schlauchleitung (7) und eine Einrichtung (22) zur Lageerkennung des mit der Schlauchführung (21) geführten Schlauchsegments (7A) aufweist, die derart konfiguriert ist, dass eine Veränderung der Lage des Schlauchsegments (7A) infolge einer Beanspruchung der Schlauchleitung (7) auf Zug erkannt wird, wobei bei einer Veränderung der Lage des Schlauchsegments auf einen fehlerhaften Gefäßzugang geschlossen wird.

2. Vorrichtung nach Anspruch 1, **dadurch gekennzeichnet, dass** die Einrichtung (22) zur Lageerkennung ein optisches Bilderfassungssystem (34) aufweist, das derart konfiguriert ist, dass eine Veränderung der Lage der Schlauchleitung (7) infolge einer Beanspruchung der Schlauchleitung auf Zug erkannt wird.

3. Vorrichtung nach Anspruch 2, **dadurch gekennzeichnet, dass** das optische Bilderfassungssystem (34) konfiguriert ist, eine Mehrzahl von beabstandeten Markierungen (36), die an der Schlauchleitung (7) angebracht sind, nacheinander einzeln zu detektieren und zu zählen, wenn die Markierungen aufgrund einer Lageänderung der Schlauchleitung (7) relativ zum Bilderfassungssystem bewegt werden und dessen Erfassungsbereich durchlaufen , wobei das optische Bilderfassungssystem (34) eine Auswerteeinheit (23) aufweist, die derart konfiguriert ist, dass die Anzahl der gezählten Markierungen (36) mit einem vorgegebenen Grenzwert verglichen wird und auf eine Veränderung der Lage der Schlauchleitung (7) infolge einer Beanspruchung der Schlauchleitung auf Zug geschlossen wird, wenn die Anzahl der gezählten Markierungen (36) größer als ein vorgegebener Grenzwert ist.

4. Vorrichtung nach Anspruch 2, **dadurch gekennzeichnet, dass** das optische Bilderfassungssystem (34) derart konfiguriert ist, dass eine Mehrzahl von beabstandeten Markierungen (36), die an der Schlauchleitung (7) vorgesehen sind, in einem Beobachtungsfenster (B) bestimmt wird, wobei das optische Bilderfassungssystem (34) eine Auswerteeinheit (23) aufweist, die derart konfiguriert ist, dass die Anzahl der Markierungen (36) in dem Beobachtungsfenster (B) mit einem vorgegebenen Grenzwert verglichen wird und auf eine Veränderung der Lage der Schlauchleitung (7) infolge einer Beanspruchung der Schlauchleitung auf Zug geschlossen wird, wenn die Anzahl der Markierungen (36) in dem Beobachtungsfenster (B) kleiner als ein vorgegebener Grenzwert ist.

5. Vorrichtung nach Anspruch 1, **dadurch gekennzeichnet, dass** die Einrichtung (22) zur Lageerkennung ein taktiles Sensorsystem (43) aufweist, das derart konfiguriert ist, dass eine Veränderung der Lage der Schlauchleitung (7) infolge einer Beanspruchung der Schlauchleitung auf Zug erkannt wird.

6. Vorrichtung nach Anspruch 1, **dadurch gekennzeichnet, dass** die Einrichtung zur Lageerkennung ein langgestrecktes Element (52) aufweist, das bei einer Verformung einen elektrischen Schalter (53) betätigt, wobei das erste Ende des langgestreckten Elements (52) mit einem ersten Abschnitt des mit der Schlauchführung (21) geführten Schlauchsegments (7A) in Wirkverbindung steht, und das zweite Ende des langgestreckten Elements (52) mit einem zweiten Abschnitt des mit der Schlauchführung (21) geführten Schlauchsegments (7A) in Wirkverbindung steht, so dass das langgestreckte Element (52) bei einer Veränderung der Lage des Schlauchsegments infolge einer Beanspruchung der Schlauchleitung auf Zug verformt wird.

7. Vorrichtung nach Anspruch 1, **dadurch gekennzeichnet, dass** die Einrichtung zur Lageerkennung ein langgestrecktes Element (54) aus einem elektrisch leitenden Material aufweist, wobei das erste Ende des langgestreckten Elements (54) mit einem ersten Abschnitt des mit der Schlauchführung (21) geführten Schlauchsegments (7A) in Wirkverbindung steht, und das zweite Ende des langgestreckten Elements (54) mit einem zweiten Abschnitt des mit der Schlauchführung (21) geführten Schlauchsegments (7A) in Wirkverbindung steht, so dass das langgestreckte Element (54) bei einer Veränderung der Lage des Schlauchsegments infolge einer Beanspruchung auf Zug durchtrennt wird, und dass die Einrichtung (22) zur Lageerkennung eine Auswerteeinheit (23) aufweist, die derart konfiguriert ist, dass der elektrische Widerstand des langgestreckten Elements (54) überwacht wird.

8. Vorrichtung nach Anspruch 1, **dadurch gekennzeichnet, dass** die Einrichtung (22) zur Lageerkennung mindestens eine Laufrolle (41) aufweist, die mit dem in der Schlauchführung (21) geführten Schlauchsegment (7A) in Kontakt bringbar ist, und einen Umdrehungsmesser (42) zum Messen der Umdrehungen der mindestens einen Laufrolle (41) aufweist, wobei eine das Messsignal des Umdrehungsmessers (42) empfangende Auswerteeinheit (23) vorgesehen ist, die derart konfiguriert ist, dass die Anzahl der Umdrehungen der Laufrolle (41) mit einer vorgegebenen Grenzwert verglichen wird.

9. Vorrichtung nach einem der Ansprüche 1 bis 8, **dadurch gekennzeichnet, dass** die Schlauchführung (21) derart ausgebildet ist, dass das Schlauchsegment (7A) in Form einer Schlaufe (33) geführt ist, die sich bei einer Zugbeanspruchung zusammenzieht.

10. Vorrichtung nach Anspruch 9, **dadurch gekennzeichnet, dass** die Schlauchführung (21) einen Gehäusekörper (30) mit einem ersten Führungskanal (31) zur Aufnahme eines ersten Abschnitts des Schlauchsegments (7A) und einem zweiten Führungskanal (32) zur Aufnahme eines zweiten Abschnitts des Schlauchsegments (7A) der Schlauchleitung (7) aufweist.

11. Vorrichtung nach Anspruch 10, **dadurch gekennzeichnet, dass** die Führungskanäle (31, 32) in einer ersten und/oder zweiten Gehäusehälfte (30A, 30B) des Gehäusekörpers (30) ausgebildet sind.

12. Vorrichtung nach Anspruch 10 oder 11, **dadurch gekennzeichnet, dass** die mindestens eine Laufrolle (41) in mindestens einem der Führungskanäle (31, 32) des Gehäusekörpers (30) vorgesehen ist.

13. Vorrichtung nach einem der Ansprüche 1 bis 8, **dadurch gekennzeichnet, dass** die Schlauchführung (21) ein bogenförmiges Führungselement (51) zur Aufnahme des Schlauchsegments (7A) aufweist, wobei das bogenförmige Führungselement (51) einen ersten bogenförmigen Abschnitt (51C) zur Aufnahme eines ersten Teilstücks des Schlauchsegments (7A) und einen zweiten bogenförmigen Abschnitt (51D) zur Aufnahme eines zweiten Teilstücks des Schlauchsegments (7A) aufweist, wobei der erste und zweite bogenförmige Abschnitt (51A, 51B) des Führungselements (51) derart beweglich miteinander verbunden sind, dass sich der Abstand zwischen den gegenüberliegenden Enden der bogenförmigen Abschnitte des Führungselements vergrößert, wenn die Schlauchleitung auf Zug beansprucht wird.

14. Vorrichtung nach einem der Ansprüche 1 bis 8, **dadurch gekennzeichnet, dass** die Schlauchführung ein bogenförmiges Führungselement (51) zur Aufnahme des Schlauchsegments aufweist, wobei das bogenförmige Führungselement (51) aus einem elastischen Material besteht, so dass sich der Abstand zwischen den gegenüberliegenden Enden der bogenförmigen Abschnitte (51A, 51B) des Führungselements (51) vergrößert, wenn die Schlauchleitung auf Zug beansprucht wird.

15. Blutbehandlungsvorrichtung mit einem extrakorporalen Blutkreislauf (I), der (i) einen zentralvenösen Katheter oder (ii) eine arterielle Schlauchleitung (6) mit einer arteriellen Punktionskanüle (5) und eine venöse Schlauchleitung (7) mit einer venösen Punktionskanüle (8) aufweist, **dadurch gekennzeichnet, dass** die Blutbehandlungsvorrichtung eine Vorrichtung (20) zur Überwachung eines Zuganges zu einem Patienten nach einem der Ansprüche 1 bis 14 aufweist.

## Claims

1. Device for monitoring access to a patient for a device by means of which a liquid is withdrawn from the patient and/or supplied to the patient via a flexible line (7), in particular for monitoring vessel access during extracorporeal blood treatment, in which blood of a patient is withdrawn from the patient via a flexible arterial line having an arterial puncture cannula and supplied to the patient via a flexible venous line having a venous puncture cannula,
**characterised in that**
the device (20) for monitoring access to a patient has a line guide (21) for guiding a line segment (7A) of the flexible line (7) and an apparatus (22) for detecting the situation of the line segment (7A) guided by means of the line guide (21), which is configured in such a way that a change in situation of the line segment (7A) due to tension applied to the flexible line (7) is detected, it being concluded that incorrect vessel access is occurring if the situation of the line segment changes.

2. Device according to claim 1, **characterised in that** the apparatus (22) for situation detection comprises an optical image capture system (34), which is configured in such a way that a change in situation of the flexible line (7) due to tension applied to the flexible line is detected.

3. Device according to claim 2, **characterised in that** the optical image capture system (34) is configured to detect a plurality of spaced-apart markings (36), applied to the flexible line (7), individually in succession, and to count when the markings are moved relative to the optical image capture system, as a result of a change in situation of the flexible line (7), and pass through the detection region thereof, the optical image capture system (34) having an evaluation unit (23), which is configured in such a way that the number of counted markings (36) is compared with a predetermined threshold and it is concluded that there is a change in situation of the flexible line (7) due to tension applied to the flexible line if the number of counted markings (36) is greater than a predetermined threshold.

4. Device according to claim 2, **characterised in that** the optical image capture system (34) is configured in such a way that a plurality of spaced-apart markings (36) provided on the flexible line (7) is determined within an observation window (B), the optical image capture system (34) having an evaluation unit (23), which is configured in such a way that the number of markings (36) in the observation window (B) is compared with a predetermined threshold and it is concluded that there is a change in situation of the flexible line (7) due to tension applied to the flexible line if the number of markings (36) in the observation window (B) is less than a predetermined threshold.

5. Device according to claim 1, **characterised in that** the apparatus (22) for situation detection has a tactile sensor system (43), which is configured in such a way that a change in situation of the flexible line (7) due to tension applied to the flexible line is detected.

6. Device according to claim 1, **characterised in that** the apparatus for situation detection has an elongate element (52), which actuates an electrical switch (53) when deformed, the first end of the elongate element (52) being in operative connection with a first portion of the line segment (7A) guided by means of the line guide (21), and the second end of the elongate element (52) being in operative connection with a second portion of the line segment (7A) guided by means of the line guide (21), such that the elongate element (52) is deformed in the event of a change in situation of the line segment due to tension applied to the flexible line.

7. Device according to claim 1, **characterised in that** the apparatus for situation detection has an elongate element (54) made of an electrically conductive material, the first end of the elongate element (54) being in operative connection with a first portion of the line segment (7A) guided by means of the line guide (21), and the second end of the elongate element (54) being in operative connection with a second portion of the line segment (7A) guided by means of the line guide (21), such that the elongate element (54) is severed in the event of a change in situation of the line segment due to applied tension, and **in that** the apparatus (22) for situation detection has an evaluation unit (23), which is configured in such a way that the electrical resistance of the elongate element (54) is monitored.

8. Device according to claim 1, **characterised in that** the apparatus (22) for situation detection has at least one roller (41), which can be brought into contact with the line segment (7A) guided in the line guide (21), and a rotation meter (42) for measuring the rotations of the at least one roller (41), an evaluation unit (23) which receives the measurement signal of the rotation meter (42) being provided and being configured in such a way that the number of rotations of the roller (41) is compared with a predetermined threshold.

9. Device according to any of claims 1 to 8, **characterised in that** the line guide (21) is formed in such a way that the line segment (7A) is guided in the shape of a loop (33), which contracts if tension is applied.

10. Device according to claim 9, **characterised in that** the line guide (21) has a housing body (30), comprising a first guide channel (31) for receiving a first portion of the line segment (7A) and a second guide channel (32) for receiving a second portion of the line segment (7A) of the flexible line (7).

11. Device according to claim 10, **characterised in that** the guide channels (31, 32) are formed in first and/or second housing halves (30A, 30B) of the housing body (30).

12. Device according to either claim 10 or claim 11, **characterised in that** the at least one roller (41) is provided in at least one of the guide channels (31, 32) of the housing body (30).

13. Device according to any of claims 1 to 8, **characterised in that** the line guide (21) has an arc-shaped guide element (51) for receiving the line segment (7A), the arc-shaped guide element (51) having a first arc-shaped portion (51C) for receiving a first sub-portion of the line segment (7A) and a second arc-shaped portion (51D) for receiving a second sub-portion of the line segment (7A), the first and second arc-shaped portions (51A, 51B) of the guide element (51) being movably interconnected in such a way that the distance between the opposite ends of the arc-shaped portions of the guide element increases when tension is applied to the flexible line.

14. Device according to any of claims 1 to 8, **characterised in that** the line guide has an arc-shaped guide element (51) for receiving the line segment, the arc-shaped guide element (51) consisting of a resilient material, such that the distance between the opposite ends of the arc-shaped portions (51A, 51B) of the guide element (51) increases when tension is applied to the flexible line.

15. Blood treatment device comprising an extracorporeal blood circuit (I) which has (i) a central venous catheter or (ii) a flexible arterial line (6) comprising an arterial puncture cannula (5) and a flexible venous line (7) comprising a venous puncture cannula (8), **characterised in that** the blood treatment device has a device (20) for monitoring access to a patient according to any of claims 1 to 14.

## Revendications

1. Dispositif de surveillance d'un accès à un patient, destiné à un dispositif avec lequel un liquide est évacué du patient et/ou amené au patient par l'intermédiaire d'une conduite en tuyau flexible (7), en particulier destiné à la surveillance de l'accès vasculaire lors d'un traitement extracorporel du sang, dans lequel le sang d'un patient est évacué du patient par l'intermédiaire d'une conduite en tuyau flexible artérielle qui présente une canule de ponction artérielle, et est amené au patient par l'intermédiaire d'une conduite en tuyau flexible veineuse qui présente une canule de ponction veineuse,
**caractérisé en ce que**
le dispositif (20) de surveillance d'un accès à un patient comprend un guide-tuyau (21) pour guider un segment de tuyau (7A) de la conduite en tuyau flexible veineuse (7), et un organe (22) de détection de la position du segment de tuyau (7A) guidé avec le guide-tuyau (21), qui est configuré de telle sorte qu'une modification de la position du segment de tuyau (7A) suite à une sollicitation en traction de la conduite en tuyau flexible (7) est détectée, un accès vasculaire défectueux en étant déduit lors d'une modification de la position du segment de tuyau.

2. Dispositif selon la revendication 1,
**caractérisé en ce que** l'organe (22) de détection de la position comporte un système optique d'acquisition d'images (34) configuré de manière à détecter une modification de la position de la conduite en tuyau flexible (7) suite à une sollicitation en traction de la conduite en tuyau flexible.

3. Dispositif selon la revendication 2,
**caractérisé en ce que** le système optique d'acquisition d'images (34) est configuré pour détecter et compter successivement et individuellement une pluralité de marquages espacés (36) fixés à la conduite en tuyau flexible (7) lorsque les marquages sont déplacés par rapport au système d'acquisition d'images, en raison d'une modification de la position de la conduite en tuyau flexible (7), et traversent sa zone de détection, le système optique d'acquisition d'images (34) comprenant une unité d'évaluation (23) configurée de telle sorte que le nombre de marquages (36) comptés est comparé à une valeur limite prédéterminée et qu'une modification de la position de la conduite en tuyau flexible (7) suite à une sollicitation en traction de la conduite en tuyau flexible en est déduite si le nombre de marquages (36) comptés est supérieur à une valeur limite prédéterminée.

4. Dispositif selon la revendication 2,
**caractérisé en ce que** le système optique d'acquisition d'images (34) est configuré de manière à définir une pluralité de marquages espacés (36), prévus sur la conduite en tuyau flexible (7), dans une fenêtre d'observation (B), le système optique d'acquisition d'images (34) comprenant une unité d'évaluation (23) configurée de manière à comparer le nombre de marquages (36) dans la fenêtre d'observation (B) à une valeur limite prédéterminée, et à en déduire une modification de la position de la conduite en tuyau flexible (7) suite à une sollicitation en traction de la conduite en tuyau flexible si le nombre de marquages (36) dans la fenêtre d'observation (B) est inférieur à une valeur limite prédéterminée.

5. Dispositif selon la revendication 1,
**caractérisé en ce que** l'organe (22) de détection de la position comporte un système de capteurs tactiles (43) configuré de manière à détecter une modification de la position de la conduite en tuyau flexible (7) suite à une sollicitation en traction de la conduite en tuyau flexible.

6. Dispositif selon la revendication 1,
**caractérisé en ce que** l'organe de détection de la position comprend un élément allongé (52) qui, en cas de déformation, actionne un interrupteur électrique (53), la première extrémité de l'élément allongé (52) étant en liaison active avec une première portion du segment de tuyau (7A) guidé par le guide-tuyau (21), et la deuxième extrémité de l'élément allongé (52) étant en liaison active avec une deuxième portion du segment de tuyau (7A) guidé par le guide-tuyau (21), de sorte que l'élément allongé (52) est déformé lors d'une modification de la position du segment de tuyau suite à une sollicitation en traction de la conduite en tuyau flexible.

7. Dispositif selon la revendication 1,
**caractérisé en ce que** l'organe de détection de la position comprend un élément allongé (54) en un matériau électriquement conducteur, la première extrémité de l'élément allongé (54) étant en liaison active avec une première portion du segment de tuyau (7A) guidé par le guide-tuyau (21), et la deuxième extrémité de l'élément allongé (54) étant en liaison active avec une deuxième portion du segment de tuyau (7A) guidé par le guide-tuyau (21), de sorte que l'élément allongé (54) est sectionné lors d'une modification de la position du segment de tuyau suite à une sollicitation en traction,
et **en ce que** l'organe (22) de détection de la position comprend une unité d'évaluation (23) configurée de manière à surveiller la résistance électrique de l'élément allongé (54).

8. Dispositif selon la revendication 1,
**caractérisé en ce que** l'organe (22) de détection de la position comprend au moins un galet de roulement (41) qui peut être mis en contact avec le segment de tuyau (7A) guidé dans le guide-tuyau (21), et comprend un compte-tours (42) pour mesurer les tours dudit au moins un galet de roulement (41), une unité d'évaluation (23) étant prévue, qui reçoit le signal de mesure du compte-tours (42) et qui est configurée de manière à comparer le nombre de tours du galet de roulement (41) à une valeur limite prédéterminée.

9. Dispositif selon l'une des revendications 1 à 8,
**caractérisé en ce que** le guide-tuyau (21) est réalisé de manière à guider le segment de tuyau (7A) sous la forme d'une boucle (33) qui se contracte lors d'une sollicitation en traction.

10. Dispositif selon la revendication 9,
**caractérisé en ce que** le guide-tuyau (21) comprend un corps de boîtier (30) présentant un premier canal de guidage (31), destiné à recevoir une première portion du segment de tuyau (7A), et un deuxième canal de guidage (32), destiné à recevoir une deuxième portion du segment de tuyau (7A) de la conduite en tuyau flexible (7).

11. Dispositif selon la revendication 10,
**caractérisé en ce que** les canaux de guidage (31, 32) sont réalisés dans une première et/ou une deuxième moitié de boîtier (30A, 30B) du corps de boîtier (30).

12. Dispositif selon la revendication 10 ou 11,
**caractérisé en ce que** ledit au moins un galet de roulement (41) est prévu dans l'un au moins des canaux de guidage (31, 32) du corps de boîtier (30).

13. Dispositif selon l'une des revendications 1 à 8,
**caractérisé en ce que** le guide-tuyau (21) comporte un élément de guidage (51) en forme d'arc destiné à recevoir le segment de tuyau (7A), l'élément de guidage (51) en forme d'arc comportant une première portion arquée (51C), destinée à recevoir un premier morceau partiel du segment de tuyau (7A), et une deuxième portion arquée (51D), destinée à recevoir un deuxième morceau partiel du segment de tuyau (7A), les première et deuxième portions arquées (51A, 51B) de l'élément de guidage (51) étant reliées l'une à l'autre de manière mobile, de telle sorte que la distance entre les extrémités opposées des portions arquées de l'élément de guidage augmente lorsque la conduite en tuyau flexible est sollicitée en traction.

14. Dispositif selon l'une des revendications 1 à 8,
**caractérisé en ce que** le guide-tuyau comprend un élément de guidage (51) en forme d'arc destiné à recevoir le segment de tuyau, l'élément de guidage (51) en forme d'arc étant constitué d'un matériau élastique, de sorte que la distance entre les extrémités opposées des portions arquées (51A, 51B) de l'élément de guidage (51) augmente lorsque la conduite en tuyau flexible est sollicitée en traction.

15. Dispositif de traitement du sang comprenant un circuit sanguin
extracorporel (I) qui comprend (i) un cathéter veineux central ou (ii) une conduite en tuyau flexible artérielle (6) ayant une canule de ponction artérielle (5) et une conduite en tuyau flexible veineuse (7) ayant une canule de ponction veineuse (8),
**caractérisé en ce que** le dispositif de traitement du sang comprend un dispositif (20) de surveillance d'un accès à un patient selon l'une des revendications 1 à 14.
